# EUROPEAN PATENT SPECIFICATION

(11) **EP 2 244 602 B1**
(45) Date of publication and mention of the grant of the patent: **13.02.2013**
(21) Application number: 09712430.9
(22) Date of filing: 20.02.2009
(51) Int. Cl.: A47B 11/00, F03G 3/00, A47F 5/025, A47C 1/14, A47C 3/18, A63J 1/02, G09B 23/06, B23Q 7/02

(54) **TURNTABLE FOR SUPPORT OF LOADED SURFACES**
DREHSCHEIBE ZUR STÜTZUNG BELADENER FLÄCHEN
PLAQUE TOURNANTE DE SUPPORT DE SURFACES CHARGÉES

(30) Priority: 22.02.2008 HU 0800122
(43) Date of publication of application: 03.11.2010
(73) Proprietor: B.B.I.P. Szellemi Tulajdonjog-hasznosito Es Vagyonkezelo Korlatolt Felelossegu Tarsasag, 1054 Budapest (HU)
(72) Inventor: BEREZNAI, József, H-1095 Budapest (HU)
(74) Representative: Köteles, Zoltan
(86) International application number: PCT/HU2009/000021
(87) International publication number: WO 2009/104033

(56) References cited:
- DE-A1- 10 337 737
- GB-A- 2 067 837
- JP-A- 2000 189 253
- SU-A1- 1 196 204
- SU-A1- 1 593 885
- SU-A1- 1 659 001
- US-A- 5 211 172
- US-A- 5 878 671
- US-B1- 6 220 394

## Description

Subject of this invention is a turntable for vertical support of loaded horizontal surfaces, that has an upper and a lower half, between which it includes a vertical rotatable guiding element.

In many cases there is a demand for rotating an object on a horizontal supporting surface so that rotation is to be slow and long-lasting, but application of any external energy sources is not required. Using electrical tools the rotation can be accomplished with any speed and within any duration, but supply or storing of electric energy is costly, requiring maintenance, and the engines themselves are very sensitive to environmental effects. Weight operated mechanical drives are well known in the prior-art, for instance from US 6 220 394 B1 or DE 103 37 737 A1.

Target of our invention is to satisfy the above demand by a simple, reliable and cheap to manufacture mechanism - a turntable - that does not require any electric or other energy from external sources, thereby it is environment-friendly and economic.

We have recognised that mechanic work performed along a small movement path evoked by gravity can be transformed to rotation so that this is performed in prolonged time interval, while make-up of energy - that is movement along the path in reverse direction - is performed faster. Pendulum-clocks work on such a principle, but there slow movement of weights causes alternating motion. Our recognition is application of a similar principle for production of rotational motion. The invention is a corresponding mechanism consisting of two halves.

The turntable according to the introductory paragraph in its most general form is such that within the guiding element a braking element is arranged that limits vertical downward movement of the loaded horizontal surface to a given speed. The guiding element includes a threaded part that converts vertical downward movement into rotational motion, where the upper half during his downward movement engaged into the threaded part, through it is connected to the lower part by mechanical coupling for rotation transfer. The braking element is configured so that - after expiration of loaded condition - it allows backward movement of the upper half relatively to the lower part practically without or with light braking, and during its downward movement the upper half is coupled to the lower part without mechanical coupling for rotation transfer.

Further the invention will be presented by drawings, where
Fig. 1 is a sectional view of the turntable according to the invention,
Fig. 2A is the enlarged braking element outlined by a sphere on Fig. 1, in a section showing upward motion,
Fig. 2B is the enlarged braking element outlined by a sphere on Fig. 1, in a section showing downward motion,
Fig. 3 is a design that is configured with an adhesive surface and that has a coupling intermediate element, in section, and
Fig. 4 is a design according to Fig. 3 in which there is an external regulating element.

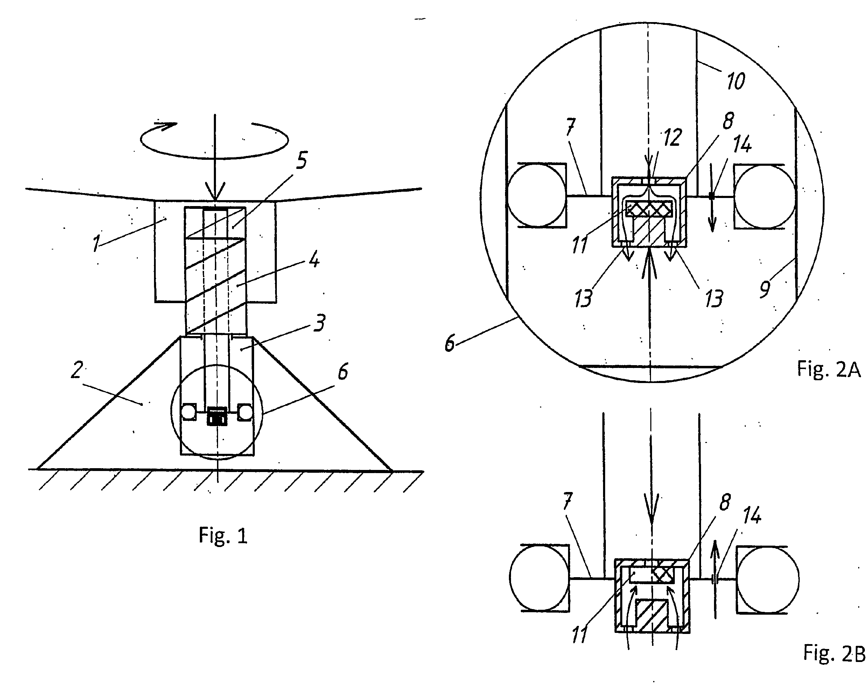

In Fig. 1 a turntable according to the invention is shown, which serves for vertical support of a practically horizontal surface. The turntable consists of two parts, of an upper' half 1 and a lower half 2 with a guiding element 3 between them that includes a threaded part 4 of fixed position relatively to the lower part 2. This threaded part 4 is a thread with high pitch with one or more starts established in the internal cavity 5 and in the inserted envelope of the upper part of the guiding element 3 as well as the connecting complementing formation. Such a complementary formation can be e.g. a full thread or only one or more protrusions from the cylindrical shell surface of the cavity 5. The essence is that in case of a force directing downwards and acting onto the upper half 1 the formation engaged into the threaded part 4, and through this establishes a mechanical coupling to the lower part 4. When it is in the condition loaded by the previous weight, under the effect of the downward pressing force the threaded part 4 with high pitch will move downward so that in the meantime rotates. We must regard to both movements - lowering and turning - as a relative displacement between the upper half 1 and the lower half 2. According to the invention, however, the task is that rotation under this gravity is performed slowly, and we can regulate even this slow speed. Our further target is that return to initial position - differently from the previous - is performed fast and without rotation. To achieve this double target, on the one hand we must establish in the guiding element 3 a braking element 6 that causes some braking of various extent against vertical movement between the lower half 1 and the upper half 2 depending on the direction. When these are approaching, the braking is large and dampens the motion to extremely slow. When they are moving away, the braking is minimal, almost zero. To accomplish this, on the exemplary design of Fig. 1, on the lower part of the guiding element 3 we show a mechanism with a piston 7 and a non-return valve 8, enlarged in Fig. 2A. In the cylinder 9 of the guiding element 3 a piston 7 is placed that air tightly seals the lower volume of the cylinder 9. Air closed here can flow outward or inward only through a valve 8. When the upper half 1 and thereby the piston arm 10 is reached and moved by a force that acts upward then the 11 sealing of the valve 8 opens, and the air can flow through bores 12, 13 along the arrow almost unobstructed into the lower closed room of the cylinder 9. This condition -that can be established by a returning element, e.g. a spring, but manual operation can also be imagined - is shown in Fig. 2A. In case of a movement in reverse direction, closed air according to Fig. 2B forced to flow outward as a result of volume decrease. This flow, however, enforces the sealing 11 to close the bore 12. Between the sealing 11 and the inner wall of the valve 8, at their contact surface we may use such solution that impairs hermetic closing, thereby allowing some low flow. This can be achieved by making the contacting surfaces roughened, abrasive or by forming at least on one of them small-sized grooves, channels, chamfers. Air diffusion ability of artificial diffusing structure worked out in such a way in contacting surfaces can be regulated. For this, previously known solutions exist and are well applicable. Alternatively, however, instead we may prepare in the piston 7 a small-sized air diffusion opening 14, e.g. a bore, which has a minimal role in the position according to Fig. 2A when the valve 8 is open, but when according to Fig. 2B the valve 8 is closed - and now we assume perfect closure - then flow-out from the closed room can be performed only through this opening 14. We can adjust the damped speed of the downward movement by the size of the opening 14 or by the total size of several openings 14. Such a design, in which we establish several small openings 14 in the piston 7, and we adjust the braking rate of the braking element 6 so that we close some of these openings 14 and release several of them. The less free openings 14 exist, the higher is damping, i.e. the slower is the achieved rotation. In the piston 7 of the braking element 6 therefore one or more passing openings 14 determine the minimum speed of diffusion of the medium based on the two directions.

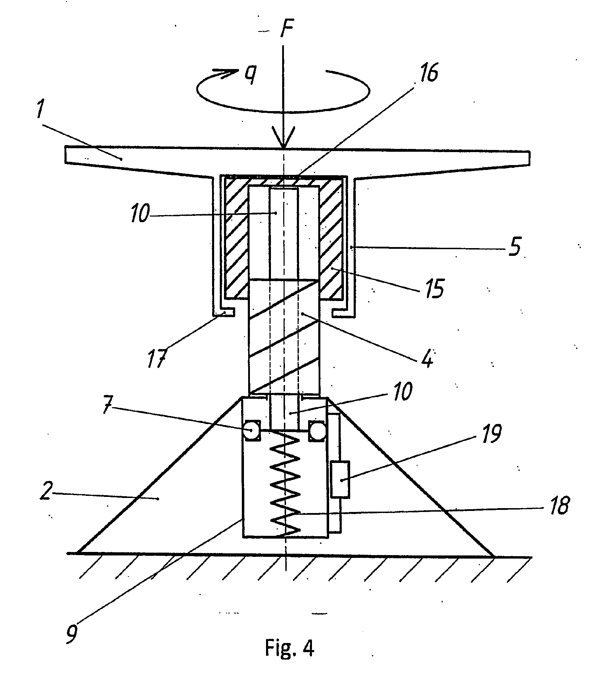

We can adjust the speed of the movement by the size of the openings 14 only as fixed. If, however, instead of borings we insert - between the lower and upper part of the cylinder 9 divided into two parts by the piston 7 - a regulating element 18 that is easily adjustable by the user then by changing the diffusing section of the regulating element the speed - and thereby the duration of lowering - becomes easily controllable. This solution is shown in Fig. 4. Here therefore we place the regulating element 19 protruding into the closed room part, in the cylinder 9 of the piston 7, so that by the regulable speed of diffusion of the medium we change the speed of lowering movement of the upper half 2. At a piston 7 moving in air medium, the other end of the regulating element 19 can be opened also into the open space. Element corresponding to the regulating element 19 - e.g. regulable valve - is a commonly known element which is easy to manufacture.

Our further object - establishment of a rotating motion during downwards movement and escaping it in reverse order - can be accomplished in several ways. One opportunity is a mechanism that engages into the threaded part 4 depending on the direction. For this, there are a number of known solutions. For this, on one of the lower half 2 and upper half 1 the threaded part 4, while on the other half the engaging mechanism is found. This part of the lower part 2 and upper part 1 practically forms concentric circles, among which the engaging mechanism constitutes the connection, but only in one direction. Formations that contact with the threaded part 4 and that are formed on the other half can be e.g. protrusions that engage into the threaded part 4. The engaging mechanism can be accomplished with these protrusions or with other similar engaging springy mechanism by a tool fixed on the thread of high pitch.

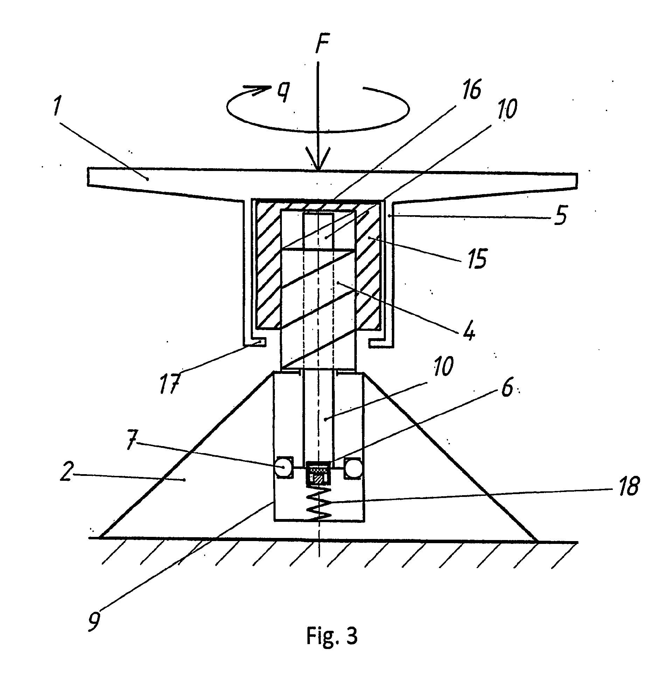

Another possible design form is shown in Fig. 3 where a part of the guiding element 3 is an intermediate element 15, which - as a screw nut mechanism - is in constant connection with the threaded part 4. Downward movement of this intermediate element 15 is limited by the braking element 6. Upper horizontal surface of the intermediate element 15 - as adhesive coupling - engages on the internal lower surface 16 of the cavity 5 formed for the upper half 1. This adhesive coupling-like engagement, however, will proceed only when a downward force is effected onto the upper half 1, therefore this presses it with the intermediate element 15. When e.g. manually or by other way we move the upper half 1 then the rotation-transferring connection established with the intermediate element 15 is broken, and the intermediate element 15 and can turn independently from the upper half 1 in the internal cavity 5 of the upper half 1, to which the lower retaining edge 17 does not transfer the rotation, it just prevents outfall of the intermediate element 15 from the upper half.

According to an expedient design, that can be seen e.g. in Fig. 3 and 4, for the braking element 6 and thereby for the upper half 1 that moves together with it, the turntable includes a returning element that in case of expiration of the loaded condition moves the braking element 6 into its upper position. The returning element expediently is a spring 18 that is supported on the lower part of the cylinder 8 and returns the piston 7 to its upper position when the load originated from gravity and exceeding the spring force expires. Instead of a returning element, or for its supplementation, manual operation can be applied additionally when we simply draw up the upper half 1.

Rotation of the piston 7 would lead to superfluous wear, therefore during its upward and downward movement it is expedient to avoid that. For this, between the piston 7 and the piston arm 10 or between the intermediate element 15 and the piston arm 10 a component decreasing friction - e.g. a bearing - can be placed.

We will describe the operation of the turntable according to the example in Fig. 3. When an object or person of sufficiently large weight is put onto the practically horizontal upper surface of the upper half 1 then it presses down that by the force F. As a result of this, the intermediate element 15 that engages into the threaded part 4 of high pitch will move downward through its full or partial inside threading, while it will turn to the rotation direction q, too. In the meantime the intermediate element 15 establishes an adhesive connection on the surface 16 of the cavity 5 according to the principle of known clutch disks, and it rotates the upper half 1 relatively to the lower part 2. Meanwhile the speed of lowering of the upper half 1, of the intermediate element 15 is determined by the braking element 6. This allows pressing-out of a given small amount of air (or liquid) due to proper valve configuration for every time unit. This limits downward movement of the piston 7, which also presses the spring 18. Movement of the piston 7 is transmitted to the upper half 1 via the piston arm 10 through the opening of the intermediate element 15. When the downward movement reaches the lower possible end position or the force F disappears earlier then the spring 18 moves the piston 7 upward, which due to the valve practically unobstructed can advance upward. Then on the surface 16 in for fail of the pressing force no adhesion is created, that is the upper half 1 advances forward without rotation, however the intermediate element 15, for which remaining in the cavity 5 is provided by the edge 17, will rotate again on the threaded part 4, while advancing upward.

Beside the described embodiments, we may also apply additional solutions to achieve braking as well as engagement to the threaded part 4 depending on the direction. Generally our target is that possible theoretical speed of loaded downward movement and unloaded upward movement - where the latter can be performed manually or with sprung return - is very different, and in most cases we wish to specify only the speed of downward movement and through the threaded part 4 thereby the speed of rotation, and returning is performed practically unobstructed, the speed or time (if it is short) of which is not important.

In the shown exemplary implementations of the braking element 6 we applied air in the space delimited by the piston 7 and the cylinder 9, but we can use liquid, too. In this case the spring effect caused by the gaseous medium falls out, and as a result of incompressibility of liquids we get a device of harder operation.

The turntable according to the invention practically serves for vertical support of horizontal surfaces and this opens various areas for application. The solution has an important advantage that no energy supply is required. Such application area can be, for example, establishment of such sunbath bed that rotates at a predetermined speed and thereby the person lying on it is turned towards the sunshine. This makes sun-tan more uniform and reduces the risk of concentrated burning of the skin. Of course, rotation speed must be adjusted so that the turnaround takes several minutes or several decades of minutes. The speed of rotation must be so low that the user does not detect it, as fast rotation can be disturbing, and may cause even dizziness, for example. For complete exclusion of risks of accidents, it is expedient to reduce also the speed of upward movement and to make the diffusing borings by observing this. With the described valve- and piston solutions, such a braking element 6 can be accomplished.

Further application areas for the turntable can be its configuration as a warehouse- or exhibition tool for product fairs.

Similarly to the previous way, we can make a maintenance-free moving advertisement tool without the need for energy supply, which requires seldom restarting only, but this may cover even a long period. Energy-free usage of the turntable according to the invention is especially advantageous in such areas (e.g. on open air) where it cannot or can be provided only in a costly way.

The turntable can be applied as a school training or demonstration tool that shows an object or phenomenon from its several sides for many people.

As a turntable, it can be configured for entertainment purposes in restaurants, at drawing-rooms, on programs.

For some mounting and working tasks, where traditionally e.g. conveyor belt can be applied, by using the turntable according to the invention, workpieces or components can be forwarded among workplaces arranged along a circle, saving energy

In bigger sizes the turntable according to the invention may be suitable also for moving of theatrical sceneries or stages.

In general, the invention is suitable for all such tasks where one or more objects must be made available or visible from different directions without additional source of energy, and this operation can be repeated after expiry of a given time interval by human intervention. The part of the turntable that establishes rotation, the guiding element can be prepared also in small size, and for this the necessary base and upper supporting surfaces, components can be inserted separately, thereby they can be easily assembled and disassembled and transported. Place of utilisation does not require any ground- or other preparation.

### Parts list

| | |
|---|---|
| 1 | upper half |
| 2 | lower half |
| 3 | guiding element |
| 4 | threaded part |
| 5 | cavity |
| 6 | braking element |
| 7 | piston |
| 8 | valve |
| 9 | cylinder |
| 10 | piston arm |
| 11 | sealing |
| 12 | boring |
| 13 | boring |
| 14 | opening |
| 15 | intermediate element |
| 16 | surface |
| 17 | edge |
| 18 | spring |
| 19 | regulating element |

## Claims

1. A turntable for vertical support of substantially horizontal loaded surfaces, which has an upper upper and a lower half of the upper half (1) relative to the lower HALF (2), between that it includes a vertically directed rotatable guiding element, **characterised in that** within the guiding element (3) a braking element (6) located, which limits the speed of the vertical downward movement of the upper horizontal surface of the upper half (1) relative to the lower HALF (2) to a given value, and the guiding element (3) includes a threaded part (4) that changes the vertical downward movement to rotating motion, where the upper half (1) during its downward movement is connected to the lower half (2) by a mechanical coupling for rotation transfer by engagement into the threaded part (4), and the braking element (6) after expiry of the loaded condition is allowing backward movement of the upper half (1) relatively to the lower half (2) substantially without braking, and where the upper half (1) during its upward movement is connected to the lower half (2) without mechanical coupling for rotation transfer.

2. A turntable according to the claim 1 **characterized in that** the braking element (6) is configured with a medium-diffusion piston (7) that is asymmetric depending on the direction.

3. A turntable according to the claim 2, **characterized in that** the asymmetric piston (7) is in a closed piston cylinder (9) filled with air or gas.

4. A turntable according to the claim 2, **characterized in that** the asymmetric piston (7) is in a closed piston cylinder (9) filled liquid.

5. A turntable to according to any of the previous claims, **characterized in that** the lower half (2) and the upper half (1) have a component part that constitute common concentric rings, among which the threaded part (4) is configured, where on one of the lower half (2) and the upper half (1) the threaded part (4), and on the other half a ratchet-closing device is formed for mechanical coupling for rotation transfer depending on the direction.

6. A turntable according to the claim 5, **characterized in that** the engaging ratchet-closing device is located in the internal cavity (5) of the lower part of the upper half (1), on its side with sprung protrusions.

7. A turntable according to any claims of 1-4, **characterized in that** the mechanical coupling for rotation transfer is accomplished on the internal lower surface (16) of the cavity (5) formed in the upper half (1), where the intermediate element (15) is engaged onto the threaded part (4) as a screw nut.

8. A turntable according to any of the previous claims, **characterised in that** it includes a returning element that in case of expiry of the loaded condition moves the braking element (6) into its upper position.

9. A turntable according to the claim 8, **characterized in that** the returning element is a spring (18).

10. A turntable according to any of the claims 2-9, **characterized in that** in the piston (7) of the braking element (6) there are one or several diffusing openings (14) that determine the lowest speed of medium diffusion depending on the direction.

11. A turntable according to any of the claims 2-10, **characterized in that** it has a regulating element (19) of medium-diffusion speed protruding into the room closed into its cylinder (9) by the piston (7).

12. A turntable according to any of the previous claims, **characterized in that** the practically horizontal loaded surface is a sunbath bed.

13. A turntable according to any of the previous claims, **characterized in that** the practically horizontal loaded surface is a warehouse tool for product fairs.

14. A turntable according to any of the previous claims, **characterized in that** the practically horizontal loaded surface is an advertisement tool.

15. A turntable according to any of the previous claims, **characterized in that** the practically horizontal loaded surface is a tool for moving of theatrical sceneries.

16. A turntable according to any of the previous claims, **characterized in that** the practically horizontal loaded surface is a school training or demonstration tool.

17. A turntable according to any of the previous claims, **characterised in that** it is configured for entertainment purposes in restaurants as an offering table.

18. A turntable according to any of the previous claims, **characterised in that** it is configured for mounting and working tasks for forwarding of workpieces or components.

## Patentansprüche

1. Drehscheibe zur senkrechten Stütze von im Wesentlichen waagerecht beladenen Flächen, die eine obere (1) und eine untere Hälfte (2) umfasst, zwischen denen sie ein senkrecht gerichtetes drehbares Führungselement umfasst, **dadurch gekennzeichnet, dass** sich in dem Führungselement (3) ein Bremselement (6) befindet, das die Geschwindigkeit der senkrechten Abwärtsbewegung der oberen waagerechten Fläche der oberen Hälfte (1) in Bezug auf die untere Hälfte (2) auf einen gegebenen Wert begrenzt, und das Führungselement (3) einen Gewindeabschnitt (4) umfasst, der die senkrechte Abwärtsbewegung zu einer Drehbewegung verändert, wobei die obere Hälfte (1) während ihrer Abwärtsbewegung durch eine mechanische Kupplung zur Drehungsübertragung durch einen Eingriff in den Gewindeabschnitt (4) mit der unteren Hälfte (2) verbunden ist, und dem Bremselement (6) nach dem Ende des beladenen Zustands eine im Wesentlichen ungebremste Rückwärtsbewegung der oberen Hälfte (1) in Bezug auf die untere Hälfte (2) gestattet ist, und wobei die obere Hälfte (1) während ihrer Aufwärtsbewegung ohne mechanische Kupplung zur Drehungsübertragung mit der unteren Hälfte (2) verbunden ist.

2. Drehscheibe nach Anspruch 1, **dadurch gekennzeichnet, dass** das Bremselement (6) mit einem Mediendiffusionskolben (7) gestaltet ist, der abhängig von der Richtung asymmetrisch ist.

3. Drehscheibe nach Anspruch 2, **dadurch gekennzeichnet, dass** sich der asymmetrische Kolben (7) in einem geschlossenen Kolbenzylinder (9) befindet, der mit Luft oder Gas gefüllt ist.

4. Drehscheibe nach Anspruch 2, **dadurch gekennzeichnet, dass** sich der asymmetrische Kolben (7) in einem geschlossenen Kolbenzylinder (9) befindet, der mit einer Flüssigkeit gefüllt ist.

5. Drehscheibe nach einem der vorhergehenden Ansprüche, **dadurch gekennzeichnet, dass** die untere Hälfte (2) und die obere Hälfte (1) einen Bestandteil aufweisen, die gemeinsame konzentrische Ringe bilden, zwischen denen der Gewindeabschnitt (4) gestaltet ist, wobei für die mechanische Kupplung zur Drehungsübertragung abhängig von der Richtung an einem aus der unteren Hälfte (2) und der oberen Hälfte (1) der Gewindeabschnitt (4) und an der anderen Hälfte eine Sperrklinken-Schließvorrichtung gebildet ist.

6. Drehscheibe nach Anspruch 5, **dadurch gekennzeichnet, dass** sich die eingreifende Sperrklinken-Schließvorrichtung in einem inneren Hohlraum (5) des unteren Teils der oberen Hälfte (1) befindet und an ihrer Seite gefederte Vorsprünge aufweist.

7. Drehscheibe nach einem der Ansprüche 1 bis 4, **dadurch gekennzeichnet, dass** die mechanische Kupplung zur Drehungsübertragung an der inneren unteren Fläche (16) des in der oberen Hälfte (1) gebildeten Hohlraums (5), wo das Zwischenelement (15) als Schraubmutter auf dem Gewindeabschnitt (4) in Eingriff gebracht ist, bewerkstelligt wird.

8. Drehscheibe nach einem der vorhergehenden Ansprüche, **dadurch gekennzeichnet, dass** sie ein Rückführungselement umfasst, das im Fall eines Endes des beladenen Zustands das Bremselement (6) in seine obere Position bewegt.

9. Drehscheibe nach Anspruch 8, **dadurch gekennzeichnet, dass** das Rückführungselement eine Feder (18) ist.

10. Drehscheibe nach einem der Ansprüche 2 bis 9, **dadurch gekennzeichnet, dass** sich in dem Kolben (7) des Bremselements (6) eine oder mehrere Diffusionsöffnungen (14) befinden, die abhängig von der Richtung die niedrigste Geschwindigkeit der Mediendiffusion bestimmen.

11. Drehscheibe nach einem der Ansprüche 2 bis 10, **dadurch gekennzeichnet, dass** sie ein Element (19) zur Regulierung der Mediendiffusionsgeschwindigkeit aufweist, das in den Raum vorspringt, der durch den Kolben (7) in dessen Zylinder (9) eingeschlossen ist.

12. Drehscheibe nach einem der vorhergehenden Ansprüche, **dadurch gekennzeichnet, dass** die praktisch waagerechte beladene Fläche eine Sonnenliege ist.

13. Drehscheibe nach einem der vorhergehenden Ansprüche, **dadurch gekennzeichnet, dass** die praktisch waagerechte beladene Fläche ein Lagerwerkzeug für Produktmessen ist.

14. Drehscheibe nach einem der vorhergehenden Ansprüche, **dadurch gekennzeichnet, dass** die praktisch waagerechte beladene Fläche ein Werbemedium ist.

15. Drehscheibe nach einem der vorhergehenden Ansprüche, **dadurch gekennzeichnet, dass** die praktisch waagerechte beladene Fläche ein Werkzeug zur Bewegung von Theaterdekorationen ist.

16. Drehscheibe nach einem der vorhergehenden Ansprüche, wobei die praktisch waagerechte beladene Fläche ein Schulübungs- oder Vorführwerkzeug ist.

17. Drehscheibe nach einem der vorhergehenden Ansprüche, **dadurch gekennzeichnet, dass** sie als Angebotstisch zu Darbietungszwecken in Restaurants gestaltet ist.

18. Drehscheibe nach einem der vorhergehenden Ansprüche, **dadurch gekennzeichnet, dass** sie zur Beförderung von Werkstücken oder Bestandteilen für Anbringungs- und Bearbeitungsaufgaben gestaltet ist.

## Revendications

1. Table tournante pe-mettant d'assurer un support vertical pour des surfaces chargées sensiblement horizontales, qui a une moitié supérieure (1) et une moitié inférieure (2) entre lesquelles elle comprend un élément de guidage rotatif dirigé verticalement, **caractérisée en ce qu'**il y a dans l'élément de guidage (3) un élément de freinage (6)', qui limite la vitesse du mouvement vertical descendant de la surface horizontale supérieure de la moitié supérieure (1) par rapport à la moitié inférieure (2) à une valeur donnée et l'élément de guidage (3) comprend une partie filetée (4) qui convertit le mouvement vertical descendant en mouvement rotatif, où la moitié supérieure (1) au cours de son mouvement descendant est raccordée à la moitié inférieure (2) par un accouplement mécanique pour un transfert en rotation par engagement dans la partie filetée (4), et l'élément de freinage (6), après expiration de l'état chargé permet un mouvement vers l'arrière de la moitié supérieure (1) par rapport à la moitié inférieure (2) sensiblement sans freinage, et où la moitié supérieure (1) au cours de son mouvement ascendant est raccordée à la moitié inférieure (2) sans accouplement mécanique pour le transfert en rotation.

2. Table tournante selon la revendication 1, **caractérisée en ce que** l'élément de freinage (6) est configuré avec un piston de diffusion d'agent (7) qui est asymétrique en fonction de la direction.

3. Table tournante selon la revendication 2, **caractérisée en ce que** le piston asymétrique (7) se trouve dans un cylindre à piston fermé (9) rempli d'air ou de gaz.

4. Table tournante selon la revendication 2, **caractérisée en ce que** le piston asymétrique (7) se trouve dans un cylindre à piston fermé (9) rempli de liquide.

5. Table tournante selon l'une quelconque des revendications précédentes, **caractérisée en ce que** la moitié inférieure (2) et la moitié supérieure (1) ont une partie constituante qui forme des anneaux concentriques communs, parmi lesquels la partie filetée (4) est configurée, où il est formé, sur l'une de la moitié inférieure (2) et de la moitié supérieure (1), la partie filetée (4) et, sur l'autre moitié, un dispositif de fermeture de cliquet pour obtenir un accouplement mécanique permettant un transfert en rotation en fonction de la direction.

6. Table tournante selon la revendication 5, **caractérisée en ce que** le dispositif de fermeture de cliquet d'engagement est situé dans la cavité interne (5) de la partie inférieure de la moitié supérieure (1), sur son côté portant des saillies encastrées.

7. Table tournante selon l'une quelconque des revendications 1 à 4, **caractérisée en ce que** l'accouplement mécanique pour le transfert en rotation est effectué sur la surface intérieure interne (16) de la cavité (5) formée dans la moitié supérieure (1), où l'élément intermédiaire (15) est engagé sur la partie filetée (4) sous la forme d'un écrou de vissage.

8. Table tournante selon l'une quelconque des revendications précédentes, **caractérisée en ce qu'**elle comprend un élément de rappel qui, dans le cas de l'expiration de l'état chargé, déplace l'élément de freinage (6) dans sa position supérieure.

9. Table tournante selon la revendication 8, **caractérisée en ce que** l'élément de rappel est un ressort (18).

10. Table tournante selon l'une quelconque des revendications 2 à 9, **caractérisée en ce qu'**il y a, dans le piston (7) de l'élément de freinage (6), une ou plusieurs ouvertures de diffusion (14) qui détermine(nt) la vitesse de diffusion de l'agent la plus basse en fonction de la direction.

11. Table tournante selon l'une quelconque des revendications 2 à 10, **caractérisée en ce qu'**elle présente un élément régulateur (19) de la vitesse de diffusion de l'agent, faisant saillie dans l'espace fermé dans son cylindre (9) par le piston (7).

12. Table tournante selon l'une quelconque des revendications précédentes, **caractérisée en ce que** la surface chargée pratiquement horizontale est un lit de banc solaire.

13. Table tournante selon l'une quelconque des revendications précédentes, **caractérisée en ce que** la surface chargée pratiquement horizontale est un outil d'entrepôt pour des salons de produits.

14. Table tournante selon l'une quelconque des revendications précédentes, **caractérisée en ce que** la surface chargée pratiquement horizontale est un outil publicitaire.

15. Table tournante selon l'une quelconque des revendications précédentes, **caractérisée en ce que** la surface chargée pratiquement horizontale est un outil pour le déplacement de décors de théâtre.

16. Table tournante selon l'une quelconque des revendications précédentes, **caractérisée en ce que** la surface chargée pratiquement horizontal est un outil de formation ou de démonstration scolaire.

17. Table tournante selon l'une quelconque des revendications précédentes, **caractérisée en ce qu'**elle est configurée à des fins de loisirs dans des restaurants sous la forme d'une table de présentation.

18. Table tournante selon l'une quelconque des revendications précédentes, **caractérisée en ce qu'**elle est configurée à des fins de montage et de travail pour l'expédition de pièces ou de composants.
